# EUROPEAN PATENT APPLICATION

(11) **EP 4 574 080 A1**
(43) Date of publication of application: **25.06.2025**
(21) Application number: 23219253.4
(22) Date of filing: 21.12.2023
(51) Int. Cl.: A61B 34/30, A61B 17/29

(54) **SURGICAL ROBOTIC SYSTEM AND APPLICATIONS AND PARTS THEREFORE**

(71) Applicant: Microsure B.V., 5692 EA Son (NL)
(72) Inventor: Boerma, Erik Niels, 5616 BS Eindhoven (NL)
(74) Representative: DTS Patent- und Rechtsanwälte PartmbB

(57) **Abstract**

The present invention provides system (200) for operating a surgical instrument (202) in an end-effector system (600) for a surgical, microsurgical or super-microsurgical robot arm, the system (200) comprising: said surgical instrument (202); at least one actuator (310) having an actuator shaft (212), and a cam system (400; 500) comprising: at least one cam (402, 404) and at least one movement system (406, 408), wherein the at least one cam (402, 404) is mounted on the actuator shaft (212), and wherein the actuator (310) is configured to actuate movement of said at least one movement system (406, 408) to operate the surgical instrument (202). The invention further provides an end-effector system (600) comprising the system (200) defined above. Still further, the invention provides a surgical, microsurgical or super-microsurgical robot arm comprising said end-effector system.

## Description

The present invention belongs to the technical field of robotic systems for use in surgery, microsurgery or super-microsurgery.

In particular, the present invention relates to a system for operating a surgical instrument in an end-effector system.

The present invention also refers to an end-effector system comprising said system.

Still further, the invention concerns a robot arm for use in surgery, microsurgery or super-microsurgery procedures.

According to a non-limiting example, the robot arm can be used to perform anastomoses.

Different kinds of surgical instruments are usually necessary when a surgical, microsurgical or super-microsurgical operation is carried out.

These surgical instruments are often configured to be manually held and manipulated by an operator, e.g., a surgeon.

Alternatively, said surgical instruments can be manipulated through a robotic system comprising one or more robot arms.

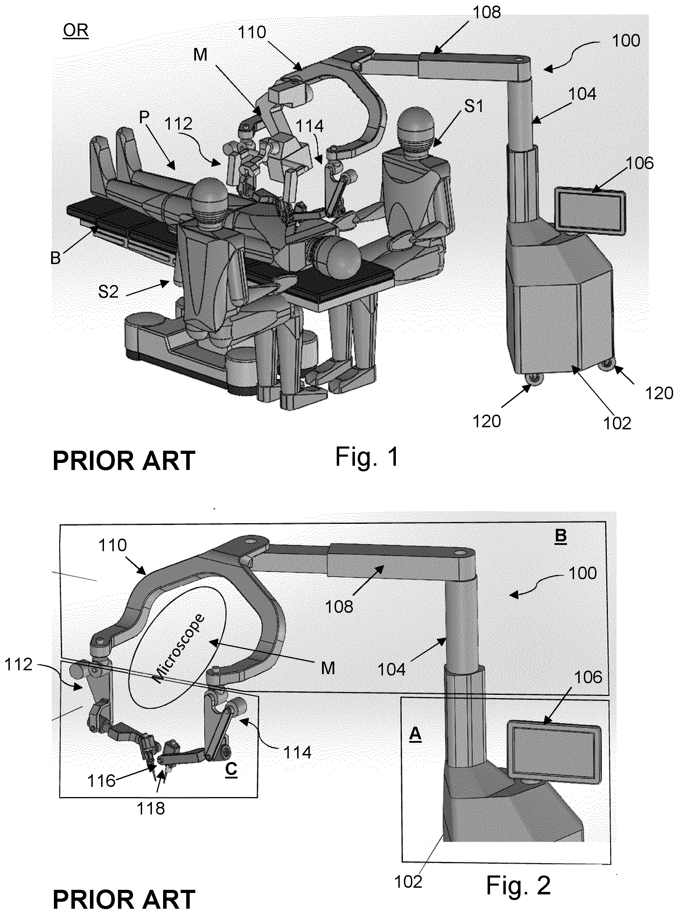

An exemplary configuration of a robotic system for use in surgery, microsurgery or super-microsurgery is shown in **Figs. 1-2****.**

The use of a robotic system facilitates accurate movements with micrometer precision without substantial tremor, thereby superseding the limitations of human hand manipulation.

Here, the use of an adapter is required to provide an interface between the surgical instrument and the end-effector of the robot arm.

Surgical instrument adapters for use in robotic systems of this kind are known in the art.

An exemplary surgical instrument adapter is described in WO2022233585A1, filed in the name of the same applicant.

In the configuration according to WO2022233585A1 (illustrated in **Fig. 3**) the surgical instrument adapter comprises a clip to at least one surgical instrument, configured to hold the at least one surgical instrument in a predefined orientation with respect to the end-effector, and to transmit a movement of a tip portion of first and second fingers of the end-effector to the at least one surgical instrument, to manipulate said at least one surgical instrument.

An improved surgical instrument adapter is disclosed in EP23207208, filed in the name of the same applicant.

Surgical, and in particular microsurgical or super-microsurgical procedures require precise and reliable manipulation of one or more surgical instruments connected to the end-effector of the robotic arm..

In particular, it is important to prevent the occurrence of stick-slip generating parasitic movement that may hinder precision and reliability at the instrument tip.

Scissors-shaped or the forceps-shape surgical instruments are often used in surgical, microsurgical or super-microsurgical procedures.

These kinds of instruments are characterized by a proximal end and a distal end, the distal end comprising two functional distal portions configured to perform a movement with respect to one another (e.g. open/close).

In scissors-shaped instruments, the two distal portions are configured to move in the same fashion as a pair of scissors.

Here, a hinge mechanism is provided in the proximity of said distal portions.

On the other hand, in forceps-shaped instruments, the two distal portions are adapted to move in the same fashion as tweezers.

Here, a hinge mechanism is rather provided at the proximal end of the instrument.

Surgical instruments of this kind are mostly designed for manual manipulation. In such cases, the hinge mechanism allows the operator, e.g. a surgeon, to conveniently hold the instrument between the index finger and the thumb, even when the distal portions of the instrument are not in contact with each other.

Both scissors-shaped and forceps-shaped instruments have in common that the two distal portions are adapted to move when corresponding proximal portions, provided at the proximal end of the instrument, are actuated.

When operating a surgical, microsurgical or super-microsurgical instrument of this kind, it is important that both distal portions of the instrument move in opposite directions by the same displacement.

Otherwise, the virtual center between the distal portions will shift in a lateral direction during movement, leading to parasitic movement that renders it difficult to grab and/or manipulate objects such as suture needles, suture wires, and/or blood vessels.

This even more applies when microsurgical or super-microsurgical procedures are concerned, requiring the highest level of precision and reliability.

In forceps-shaped instruments, the distal portions move at a magnified rate compared to the movement of the corresponding proximal portions.

This may lead to a correspondingly magnified parasitic movement.

In robotic surgery, microsurgery or super-microsurgery, the instrument, e.g. a scissors-shaped or a forceps-shaped instrument, is operated through an actuator, e.g. an electric motor.

Two main approaches are known in the art to operate scissors-shaped or forceps-shaped surgical instruments, in particular to control the opposite, equal movement of the distal portions of the instrument.

According to a first approach, an actuator, e.g. an electric motor, can be provided for each distal portion of the instrument.

Here, both actuators are controlled through a control mechanism, so that movements of the distal portions can be compared and, if deviation is detected, correspondingly adjusted through the control mechanism to prevent (or at least reduce) the occurrence of parasitic movement.

This first approach has the drawback that the overall weight, volume and energy consumption of the robot arm is increased, in particular as a consequence of the provision of two actuators.

A second approach contemplates the use of a single actuator, e.g. an electric motor.

In particular, the actuator is mechanically and operatively connected to both distal portions of the scissors-shaped or forceps-shaped instrument, to actuate and control the opposite movement of the distal portions.

Here, no control mechanism is required to adjust for parasitic movement

This second approach has proven advantageous in particular in that it requires fewer mechanical components, thereby allowing reducing the overall weight and volume of the robot arm, further than heat generation, computational effort and/or risks of failure (in principle, the higher the number of mechanical components, the higher the potential risks of failures).

These aspects are particularly relevant in robotic surgical, microsurgical or super-microsurgical systems, where the implemented robotic solution heavily depends on the dimensions of the most distal unit of the robot arm, i.e. the end-effector.

In principle, there are no particular challenges in providing a single actuator, e.g. an electric motor, for controlling two portions moving towards each other.

Nonetheless, the situation becomes more complex in case movements of said portions need to be around a fixed virtual midpoint, as in the case of a scissors-shaped or forceps-shaped instruments for use in robotic surgery, microsurgery or super-microsurgery.

Here, when the displacement of the two distal ends of the instrument is not equal (and opposing), this causes a shift in the virtual midpoint.

Also, particularly when displacing the distal ends on micro level, non-movement of one of the distal ends causes a shift in the virtual midpoint.

Stick-slip occurring in different magnitude in at least one of the distal portions of the instrument will generate unwanted parasitic movement, which is prejudicial to precision at the instrument tip.

Especially when the distal ends of the instrument are touching, and forces applied to a foreign body such as a surgical suture needle are increased, unexpected movement of the virtual midpoint can cause difficulty completing the surgical task.

As an example, when closing a dilator, a shifting mid-position makes it hard to place it in the vessel, or when opening close to the touching point, it may move out of the vessel because of the shift.

Also, stick-slip with equal or comparable magnitude on the opposing distal ends is degrative to the usability for microsurgery or super-microsurgery procedures.

In particular, this may cause a performed movement to be less smooth, or - at micro level - even to be seen as intermittent.

Especially when operating close to the touching point of the distal ends of the instrument, the intermittent displacement can be larger, or even significantly larger, than the intended displacement.

In particular, systems comprising sliding parts have the tendency to show parasitic movement of this kind.

Most available industrial grippers rely on sliding parts either in their movement or actuation.

Therefore, there is the need for an improved solution enabling a smoother manipulation of a surgical, microsurgical or super micro-surgical instrument, e.g. a scissors-shaped or forceps-shaped instrument, able to prevent the occurrence of stick-slip causing parasitic movement that may hinder precision at the instrument tip.

Also, there is the need for a solution having a simplified structure, which allows reducing the overall weight, volume and energy consumption of the robot arm, at the same time reducing the risk of failures.

In the light of the above, it is an object of the present invention to provide an improved system for operating a surgical instrument in an end-effector system for a surgical, microsurgical or super-microsurgical robot arm, capable of effectively preventing the occurrence of stick-slip causing parasitic movement that may hinder precision at the instrument tip.

Another object of the invention is to provide a solution having a simplified structure, allowing reducing weight, volume and energy consumption of the robot arm, further preventing the risk of failures.

The above-specified object is achieved by the provision of a system for operating a surgical instrument as defined in claim 1.

According to the invention, a system for operating a surgical instrument in an effector system for a surgical, microsurgical or super-microsurgical robot arm is provided, said system at least comprising:
said surgical instrument;
at least one actuator having an actuator shaft, and
a cam system comprising:
   at least one cam and
   at least one movement system,

wherein the at least one cam is mounted on the actuator shaft, and
wherein the actuator is configured to actuate movement of the at least one movement system to operate the surgical instrument.

The present invention provides a system for operating a surgical instrument in an end-effector system.

In particular, the end-effector system is adapted for use in a surgical, microsurgical or super-microsurgical robot arm.

As a non-limiting example, the robot arm can be adapted to perform anastomoses.

The system comprises a surgical instrument.

The system further comprises at least one actuator, said at least one actuator having an actuator shaft.

There is also a cam system provided in the system.

In particular, the cam system comprises at least one cam.

The cam system further comprises at least one movement system.

The at least one cam is mounted on the actuator shaft.

The actuator is configured to actuate movement of the at least one movement system, to operate the surgical instrument.

The invention is based on the basic idea that, by the provision of a cam system as defined above, a surgical instrument, mounted to the end-effector, can be manipulated in a smoother and more precise manner, preventing the occurrence of stick-slip causing parasitic movement that may hinder precision and reliability at the surgical instrument tip.

This is even more relevant in microsurgical or super-microsurgical procedures, e.g. anastomoses, where the highest level of precision and reliability is required.

Another advantage of the invention is that the structure of the end-effector can be simplified, thereby reducing the overall weight, volume and energy consumption of the robot arm.

This latter aspect is even more apparent when a single actuator and corresponding cam system are provided, which allows significantly reducing the overall number of mechanical components that are present in the system.

The provision of a simplified structure further allows preventing the potential risk of failures.

Preferably, the cam system comprises a first cam and a second cam.

Both the first cam and the second cam are mounted on the actuator shaft.

The first cam and the second cam are arranged in different planes.

This arrangement is required when two (or more) cams are provided, to prevent each roller from coming into contact with a cam track other than the cam track of its corresponding cam.

The first cam and the second cam are arranged displaced by a predefined angle with respect to one another about the rotational axis of the actuator shaft.

For instance, the first cam and the second cam may be displaced by an angle different than 180° with respect to one another about the rotational axis of the actuator shaft.

Advantageously, the first cam and the second cam have the same profile.

The placement of rollers and motor shaft is preferably not on one line to avoid play in the rollers to cause a jump.

The surgical instrument may have a proximal end and a distal end, the distal end of the instrument comprising a first functional distal portion and a second functional distal portion.

The first and second distal portions may be configured to switch between a closed position and an open position with respect to one another.

The first and second distal portions of the surgical instrument may be connected through a hinge mechanism.

The hinge mechanism may also allow movement (e.g. open/close) of the distal portions with respect to one another.

As a non-limiting example, the surgical instrument can be a scissors-shaped or a forceps-shaped surgical instrument. In this configuration, the at least one cam may comprise a first section and a second section.

In particular, the first section is characterized by a first profile that allows for a large, less precise movement of the first and second distal portions of the surgical instrument.

In particular, the second section is characterized by a second profile that allows for a precise and strong movement of the first and second distal portions of the surgical instrument.

Accordingly, it is possible to implement a different actuation on a single mechanism in the light of the surgical, microsurgical or super-microsurgical task at hand.

In particular, parameters for the at least one actuator can be changed without changing the overall working principle of the system.

The available motor torque is the same over the entire rotation of the cams.

The torque multiplied by the rotated angle results in power.

This is the same for all angle increments.

On a larger incline section, a small angle increase determines a large movement of the instrument tips, but a smaller force.

On the other hand, on a lower incline section, a small angle increase determines a small movement, combined with a high force.

The at least one movement system may comprise at least a first roller and a second roller.

In this configuration, the provision of first and second cams displaced by a predefined angle, preferably different than 180°, and having the same profile allows facilitating smooth movement of the first and second rollers by the same distance, in opposite directions.

The first roller may be mounted on a corresponding first lever while the second roller may be mounted on a corresponding second lever, to control displacement of the first and second distal portions of the surgical instrument, e.g. a scissors-shaped or a forceps-shaped surgical instrument, respectively.

Conveniently, the actuator may be configured to actuate movement of the first and second rollers by the same displacement, in opposite directions.

As a consequence, the first and second distal portions of the surgical instrument are correspondingly moved by the same distance in opposite directions, through the first and second levers.

This allows achieving smooth and precise operation of the surgical instrument, preventing the occurrence of stick-slip generating unwanted parasitic movement.

The first and second rollers may be arranged on opposite sides of the at least one cam, preferably in a mirrored position with respect to each other.

The system may be further implemented with a third cam and a corresponding third roller.

This allows reducing the load on the actuator, e.g. an electric motor motor.

In particular, the provision of the third cam and corresponding roller allows providing an additional opening force at the most open position, which does no longer need being provided by the actuator.

Accordingly, current draw and temperature increase can be reduced.

This may potentially allow for a reduced actuator size.

The first cam, the second cam and the third cam are arranged in different planes.

As mentioned, this allows preventing each roller from coming into contact with a cam track other than the cam track of its corresponding cam.

Preferably, the third cam has its incline in the opposite direction with respect to the incline of the first and second cams.

Advantageously, the third roller is arranged in the same plane as the third cam.

The spring force pressing the third roller against the corresponding third cam results in a torque around the actuator shaft having the largest magnitude at the point where the load from the instrument is the highest.

This reduces the maximum torque on the actuator, resulting in lower energy use and/or heat generation.

This potentially allows for use of a smaller actuator, e.g. an electric motor.

The system can be fitted with a spring pulling the rollers together, thereby helping opening an instrument further than its internal spring allows.

As a drawback, however, this also increases the load on the actuator, e.g. an electric motor, especially at the closing end of the movement.

The third roller can compensate for this extra load.

In this way, the system is capable of pulling open an instrument wider, without reducing the maximum closure force. The system may comprise a first actuator in combination with first cam system and a second actuator in combination with a second cam system.

That is, the system may be provided with two actuators with corresponding cam systems.

The solution has proven effective to enable smooth and precise operation of the surgical instrument, although at the expense of structural simplification.

In particular, the provision of two actuators and respective cam systems necessarily increases the overall weight, volume and energy consumption of the robot arm.

Conveniently, the rotational axis of the at least one cam and the rotational axis of the rollers are parallel to each other.

The end-effector system also comprises a sterile drape.

In particular, the sterile drape is configured to cover the end-effector, e.g. in the same fashion as a glove.

There is also a surgical instrument adapter provided in the end-effector system.

In particular, the surgical instrument adapter is configured to transmit a movement of a tip portion of the first and second movable fingers of the end-effector to the at least one surgical instrument to be operated through the robot arm.

Still further, the present invention provides a surgical, microsurgical or super-microsurgical robot arm.

The robot arm comprises an end-effector system defined above.

In a non-limiting example, the robot arm may be adapted to perform anastomoses.

Further advantages of the present invention shall now be disclosed in connection with the drawings, where:
- **Fig. 1**: shows a robotic system for use in surgery, microsurgery or super-microsurgery according to the prior art, in an operative state;
- **Fig. 2**: shows a detail of the surgical robotic system of **Fig. 1****,** where different sections of the robotic system, respectively denoted with letters **A, B, C,** are identified;
- **Fig. 3**: shows an end-effector system provided with a surgical instrument adapter according to the prior art, in particular as described in WO2022233585A1. Here, a surgical instrument is mounted to the adapter;
- **Fig. 4**: shows a system for operating a surgical instrument according to a first embodiment of the invention. Here, a single cam system comprising first and second cams, in particular radial cams, is provided. Not visible in the figure is that the system further comprises a single actuator, e.g. an electric motor;
- **Fig. 5**: shows a detail of the cam system in the configuration of **Fig. 4****,** in an operative state;
- **Fig. 6**: shows an end-effector system according to an embodiment of the invention. The end-effector system includes, inter alia, the system of **Fig. 4****.** Here, the end-effector system comprises a surgical instrument adapter as described in EP23207208;
- **Fig. 7**: shows another configuration of the system of **Fig. 4****.** Here, the system is further implemented with a third cam, in particular a radial cam;
- **Fig. 8**: shows yet another configuration of the system of **Fig. 4****.** Here, the system is implemented with two actuators and corresponding cam systems;
- **Fig. 9**: shows a system for operating a surgical instrument according to a second embodiment of the invention. In particular, the shown system is similar to that of **Fig. 7****,** but is characterized by a stacked configuration.

**Fig. 1** shows an example of a robotic system for use in surgery, microsurgery or super-microsurgery.

Said exemplary system is denoted with 100.

Here, the system 100 is shown in an operating state.

A first surgeon S1 and a second surgeon S2 are located at a respective side of an operating bed B to perform a surgical, microsurgical or super-microsurgical operation on a patient P, laying on operating bed B.

The robotic system 100 includes a base station 102.

The surgical robotic system 100 further includes a base column 104, here in the shape of a cylindrical pillar.

In the shown example, the base station 102 conveniently includes a display module 106 for displaying information to one or more operators, e.g. the surgeons S1, S2 or other personnel that is present in the operating room OR.

Also, the display module 106 may be used to define a user interface allowing one or more operators to provide a user input.

The base station 102 comprises a plurality of wheels 120 for ease of placement of the robotic system 100 at a desired location within the operating room OR.

In the shown example, the robotic system 100 is equipped with a suspension arm 108, a fork-like element 110, a first surgical, microsurgical or super-microsurgical robot arm 112, and a second surgical, microsurgical or super-microsurgical robot arm 114.

Here, the suspension arm 108 is horizontally arranged.

Also, the suspension arm 108 has two parts allowing to adjust its length by telescopically moving said parts relatively to each other.

It is also possible that a different means is used for length adjustment of the suspension arm 108, e.g. a SCARA-mechanism, hinge mechanisms, or the like.

The suspension arm 108 is connected to the base column 104.

The suspension arm 108 carries the fork-like element 110 (e.g. a bracket).

As shown in detail in **Fig. 2****,** the fork-like element 110 carries the first surgical, microsurgical or super-microsurgical robot arm 112 and the second surgical, microsurgical or super-microsurgical robot arm 114.

Each of the first and second robot arms 112, 114 is connected to an end-effector system carrying a surgical instrument 116, 118 (**Fig. 2**).

The surgical instruments 116, 118 may be of the same kind, or may be different from each other.

The suspension arm 108 is configured to rotate about the longitudinal axis of the base column 104, to allow desired positioning of the fork-like element 110 and the robot arms 112, 114 with respect to the surgical site.

Similarly, the fork-like element 110 is configured to rotate about an axis of the suspension arm 108, i.e. parallel to the longitudinal axis of the base column 104.

The robotic system 100 may be adapted for use with a conventional microscope M, as shown in **Fig. 2****.**

Alternatively, a fully digital microscope (not shown) or a so-called hybrid microscope (not shown) can be used. In such a case, one or more surgeons will be located at a separate console, and a robot trolley will be located at the position of one of the surgeons S1, S2 shown in **Fig. 1****.**

The surgical robotic system 100 provides a large patient side setup, this meaning that no assembly is required after draping.

**Fig. 2** shows different sections A, B, C of the surgical robotic system 100.

In section A, there is no movement and everything is thus stationary.

The display means 106 (included in section A) can be activated by one or more operators.

In section B, there is some movement during the surgery, in order to correctly reposition the surgical instruments 116, 118 over the patient P.

As shown in **Fig. 2****,** a space is defined between the surgical robotic arms 112, 114 for a microscope (not illustrated but only symbolized in **Fig. 2**).

Section C includes, inter alia, the end-effector.

Here, several movements occur in the course of the surgical procedure.

In this context, the functions of the end-effector are, inter alia, to actuate the surgical instruments 116, 118, provide a mounting interface, and rotate around the instrument axis.

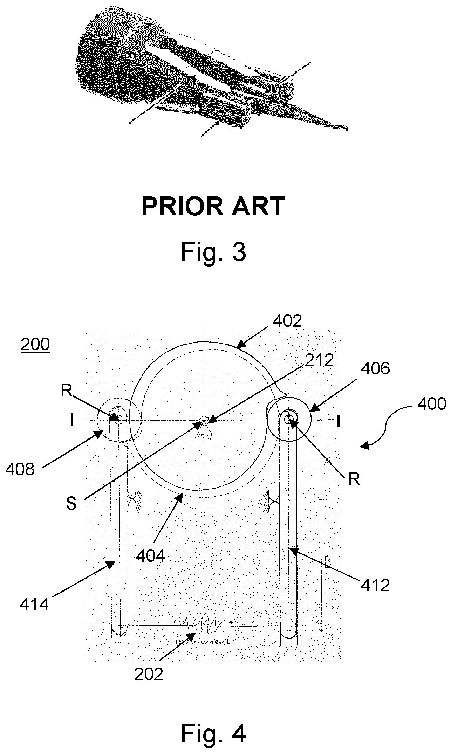

**Fig. 4** shows a system 200 for operating a surgical instrument 202 according to an embodiment of the invention.

The system 200 is adapted for use in an end-effector system for a surgical, microsurgical or super-microsurgical robot arm, such as the end effector system 600 described below.

As a non-limiting example, the robot arm may be adapted to perform anastomoses.

In the present embodiment, the system 200 includes a single actuator (not shown in the drawings).

The provision of a single actuator is convenient as it allows simplifying the structure of the end-effector, in turn resulting in a reduction of the overall weight, volume and energy consumption of the robot arm.

Also, due to the reduced number of mechanical components, the risk of failures can be effectively prevented.

As a non-limiting example, the actuator can be an electric motor.

The system 200 further comprises a cam system 400.

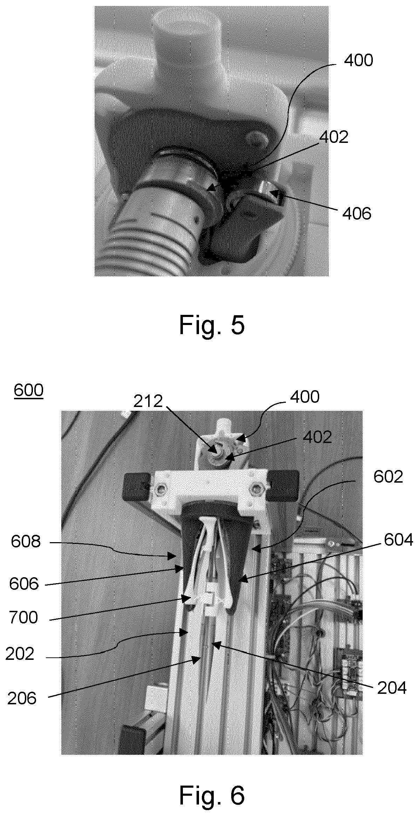

**Fig. 5** shows a detail of the cam system 400 in an operative state.

In the shown embodiment, the cam system 400 comprises a first cam 402 and a second cam 404 **(****Fig. 4****).**

In the shown embodiment, the first cam 402 and the second cam 404 are radial cams.

Not shown is that the first cam 402 and the second cam 404 may as well be barrel cams.

Not shown is also that the cam system 400 may as well comprise only a single cam, without prejudice to its functionality.

The cam system 400 also comprises at least one movement system 406, 408.

In the shown embodiment, the at least one movement system 406, 408 comprises a first roller 406 and a second roller 408.

The system 200 further comprises a surgical instrument 202.

In the present embodiment, as shown in **Fig. 6****,** he surgical instrument 202 comprises a proximal end and a distal end.

In particular, the distal end of the instrument 202 comprises a first functional distal portion 204 and a second functional distal portion 206.

The first and second distal portions 204, 206 of the surgical instrument 202 are configured to move with respect to one another.

In particular, the first and second distal portions 204, 206 of the surgical instrument 202 may be adapted to switch between a closed position and an open position with respect to one another.

The first and second distal portions 204, 206 of the surgical instrument 202 may be connected to one another through a hinge mechanism.

As a non-limiting example, the surgical instrument 202 may be a scissors-shaped or a forceps-shaped surgical instrument.

As mentioned, surgical instruments of this kind are mostly adapted for manual manipulation. In this case, the presence of a hinge mechanism, such as the hinge mechanism , allows the operator, e.g. a surgeon, to conveniently hold the instrument between the index finger and the thumb, even when the first and second distal portions of the instrument are not in contact with each other.

In the solution according to the invention, the spring mechanism leads to an always present positive contact force between each of the rollers 406, 408 and the respective cam 402, 404, .

This is advantageous since the cams 402, 404, do not need to be double-sided.

This allows achieving a further structural simplification.

Not shown is that the system 200 may be also used with surgical instruments of a different kind.

The first cam 402 and the second cam 404 are mounted on the actuator shaft 212.

In the shown embodiment, the first roller 406 and the second roller 408 are arranged on opposite sides of the cams 402, 404.

In particular, in this configuration, the first and second rollers 406, 408 are arranged mirrored with respect to one another along axis I-I (**Fig. 4**). In the present embodiment, the first roller 406 is mounted on a first lever 412, while the second roller 408 is mounted on a second lever 414.

This allows controlling displacement of the first and second distal portions 204, 206 of the surgical instrument 202, respectively.

In particular, according to the invention, the actuator is configured to actuate movement of the first and second rollers 406, 408 by the same displacement in opposite directions.

Therefore, the first and second distal portions 204, 206 of the surgical instrument 202 are correspondingly moved by the same distance in opposite directions, through the first and second levers 412, 414.

Not shown is that different arrangements of the movement system 406, 408, e.g. the first and second rollers 406, 408 illustrated in **Fig. 4****,** are possible without prejudice to the functionality of the system 200.

By the provision of a cam system 400 as described above, it is possible to operate the surgical instrument 202, e.g. a scissors-shaped or a forceps-shaped surgical instrument, smoothly and with enhanced precision.

In particular, stick-slip at the instrument tip can be prevented, reducing the occurrence of unwanted parasitic movement that may hinder precision at the instrument tip.

The first cam 402 and the second cam 404 are arranged in different planes, such that each roller 406, 408 can be prevented from contacting a cam track other than the cam track of its respective cam 404, 406.

The first cam 402 and the second cam 404 are arranged displaced with respect to one another by a predefined angle about the rotational axis S of the actuator shaft 212.

In particular, in the shown embodiment, the first cam 402 and the second cam 404 are displaced by an angle different than 180° with respect to one another about the rotational axis S of the actuator shaft 212 (**Fig. 4**).

In particular, this feature allows achieving an improved adjustment for parasitic a movement caused by the rollers 406, 408 that are not moving over a linear path.

In the shown embodiment, the first cam 402 and the second cam 404 have the same profile.

Not shown is that the first and second cams 402, 404 may also have a slightly different profile and/or dimensions, without prejudice to the functionality of the system 200.

Conveniently, in the present embodiment, each of the first cam 402 and the second cam 404 includes a first section and a second section.

In particular, the first section is characterized by a first profile that allows for a large, less precise movement of the first and second distal portions 204, 206 of the surgical instrument 202.

The second section is characterized by a second profile that allows for a precise and strong movement of the first and second distal portions 204, 206 of the surgical instrument 202.

In the present embodiment, the rotational axis S of the at least one cam 402; 404 and the rotational axis R of the rollers 406, 408 are parallel to each other.

This has the advantage that variations/tolerances at the tip of the instrument 202 can be reduced, further preventing the occurrence of unwanted parasitic movement.

This also allows for an easier manufacturing of the system 200.

**Fig. 6** shows an end-effector system 600 comprising the system 200 shown in **Fig. 4****.**

The end-effector system 600 also comprises an end-effector 602.

The end-effector 602 comprises an end-effector base (not visible in **Fig. 6**).

The end effector 602 further comprises a first movable finger 604 and a second movable finger 606, distally extending from the end-effector base.

Preferably, the first and second movable fingers 604, 606 are connected to one another through a hinge.

There is also a sterile drape 608 provided in the end-effector system 600.

In particular, the sterile drape 608 is configured cover the end-effector 602, e.g. in the same fashion as a glove.

Still further, the end effector system 600 comprises a surgical instrument adapter 700.

In particular, the surgical instrument adapter 700 is configured to transmit a movement of a tip portion of the first and second movable fingers 604, 606 of the end-effector 602 to the at least one surgical instrument 202 to be operated through the robot arm.

The end-effector system 600 is adapted for use with a surgical, microsurgical or super-microsurgical robot arm.

As a non-limiting example, the robot arm can be used to perform anastomoses.

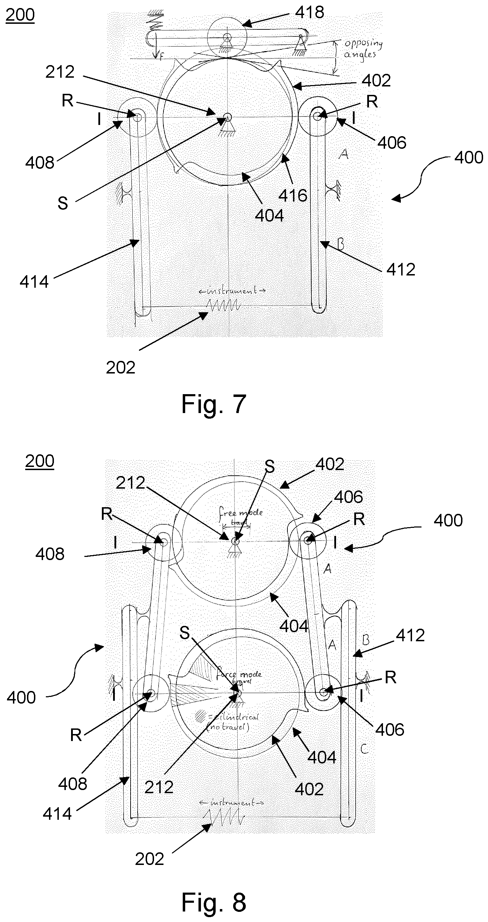

**Fig. 7** shows a further configuration of the system 200 according to the invention.

Here, the cam system 400 further comprises a third cam 416 and a corresponding third roller 418.

The first cam 402, the second cam 404 and the third cam 416 are arranged in different planes.

Furthermore, the third cam 416 has its incline in the opposite direction with respect to the incline of the first and second cams 402, 404.

In the shown configuration, the first cam 402, the second cam 404 and the third cam 416 have the same profile.

Not shown is that the cams 402, 404 may also have a slightly different profile and/or dimensions, without prejudice to the functionality of the system 200.

Not shown is that the cam 416 may also have a different profile and/or dimensions.

Here, the third roller 418 is arranged above the third cam 416.

The addition of the third cam 416 and corresponding roller 418 allows reducing the load on the actuator, e.g. an electric motor.

In particular, the provision of the third cam 416 and corresponding roller 418 allows providing an additional opening force at the most open position, which therefore does no longer need being provided by the actuator, e.g. an electric motor.

Accordingly, current draw and temperature increase can be reduced.

This may potentially allow for a reduced actuator size..

**Fig. 8** shows yet another configuration of the system 200 according to the invention.

Here, the system 200 includes a first actuator in combination with a first cam system 400 and a second actuator in combination with a second cam system 400.

Otherwise stated, the system 200 comprises two actuators, each provided with a respective cam system 400.

In the shown configuration, the first cam system 400 and the second cam system 400 are identical, and include the same features of the cam system 400 shown in **Fig. 4****.**

This configuration allows obtaining smooth and reliable robotic manipulation of the instrument, although at the expense of structural simplification.

The cams 402, 404 of at least one cam system 400 may have a shallow slope and a cylindrical segment.

At this cylindrical segment, the motor torque required to maintain position is zero.

It can be used to superimpose fine adjustment to the displacement forced on the instrument 202.

In this configuration, one actuator can be used for actuating large displacement, while the other actuator can be used for fine positioning.

As mentioned, the actuators can be electric motors.

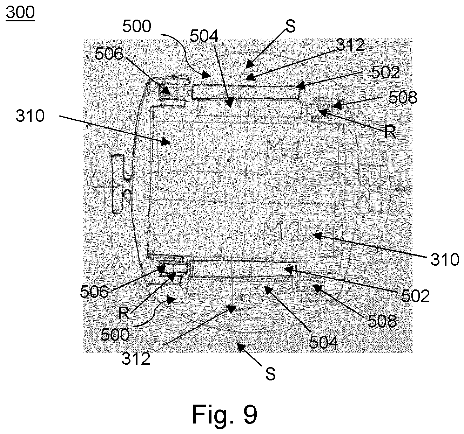

**Fig. 9** shows a system 300 for operating a surgical instrument according to a second embodiment of the invention.

The structural and functional features of the system 300 of the present embodiment are similar to that of the system 200 described above.

Also, the system 300 may be adapted for use in an end-effector system, such as the end-effector system 600 shown in **Fig. 6****.**

In principle, the system 300 according to the present embodiment differs from the system 200 described in the foregoing in that is has a stacked configuration.

The system 300 comprises a surgical instrument (not shown), such as the above-described surgical instrument 202 shown in **Fig. 6****.**

In the shown embodiment, the system 300 comprises two actuators 310, e.g. electric motors, having an actuator shaft 312.

A cam system 500 is provided for each actuator 310 (**Fig. 9**).

In the shown embodiment, each cam system 500 comprises a first cam 502 and a second cam 504 In the shown embodiment, similar as above, the first and second cams 502, 504 are radial cams.

Not shown is that the first and second cams 502, 504 may as well be barrel cams.

The first and second cams 502, 504 are mounted on the actuator shaft 312.

The cam system 500 also comprises at least one movement system 506, 508.

In the present embodiment, similar as above, said at least one movement system 506, 508 comprises a first roller 506 and a second roller 508.

In the shown configuration, the movement of each roller 506, 508 on a respective cam 502, 504 causes their axis of rotation to be non-parallel to that of the cam motion for most of the trajectory.

In the shown configuration, the first and second rollers 506, 508 are arranged on opposite sides of the first and second cams 502, 504, mirrored with respect to each other.

The first and second rollers 506, 508 are mounted on a respective lever (not shown in **Fig. 9**), to control displacement of the first and second distal portions of the surgical instrument, e.g. the first and second distal portions 204, 206 of the surgical instrument 202 shown in **Fig. 6****.**

The functioning of rollers 506, 508 is similar to that of rollers 406, 408 of the system 200, and is thus not further described for the sake of brevity.

Not shown is that different arrangements of the movement system 506, 508, e.g. the first as second rollers 506, 508 illustrated in **Fig. 9****,** are possible without prejudice to the functionality of the system 300.

Not shown is that the cam system 500 may as well include a single cam, without prejudice for its functionality.

Also not shown is that the system 300 may be configured to include a single actuator 310 and a single cam system 500.

This allows obtaining an overall simplified configuration, resulting in a reduction of weight, volume and energy consumption of the robot arm.

The functioning and the advantages of the system 300 according to the present embodiment are the same described above with respect to the system 200 of the first embodiment, and are therefore not further described for the sake of brevity.

### Reference list

- 100: Robotic system
- 102: Base station
- 104: Base column
- 106: Display module
- 108: Suspension arm
- 110: Fork-like element
- 112: (First) surgical, microsurgical or super-microsurgical robot arm
- 114: (Second) surgical, microsurgical or super-microsurgical robot arm
- 116: Surgical instrument
- 118: Surgical instrument
- 120: Wheels

- 200, 300: System for operating a surgical instrument
- 202: Surgical instrument
- 204: First functional distal portion
- 206: Second functional distal portion
- 310: Actuator
- 212; 312: Actuator shaft

- 400; 500: Cam system
- 402; 502: First cam
- 404; 504: Second cam
- 406; 506: First roller
- 408; 508: Second roller
- 412: First lever
- 414: Second lever
- 416: Third cam
- 418: Third roller

- 600: End-effector system
- 602: End-effector
- 604: First movable finger
- 606: Second movable finger
- 608: Sterile drape

- 700: Surgical instrument adapter

- B: Operating bed
- M: Microscope
- S1: First surgeon
- S2: Second surgeon
- P: Patient
- OR: Operating room

- S: Rotational axis (actuator shaft)
- R: Rotational axis (roller)

## Claims

1. A system (200; 300) for operating a surgical instrument (202) in an end-effector system (600) for a surgical, microsurgical or super-microsurgical robot arm, the system (200; 300) comprising:
said surgical instrument (202);
at least one actuator (310) having an actuator shaft (212; 312), and
a cam system (400; 500) comprising:
at least one cam (402, 404; 502, 504), and
at least one movement system (406, 408; 506, 508),
wherein the at least one cam (402, 404; 502, 504) is mounted on the actuator shaft (212; 312), and
wherein the actuator (310) is configured to actuate movement of the at least one movement system (406, 408; 506; 508) to operate the surgical instrument (202).

2. The system (200; 300) according to claim 1,
**characterized in that**
the cam system (400; 500) comprises a first cam (402; 502) and a second cam (404; 504), arranged in different planes.

3. The system (200; 300) according to claim 2,
**characterized in that**
the first cam (402; 502) and the second cam (404; 504) are arranged displaced with respect to one another by a predefined angle about the rotational axis (S) of the actuator shaft (212, 312),
preferably wherein the first cam (402; 502) and the second cam (404; 504) are arranged displaced by an angle different than 180° with respect to one another about the rotational axis (S) of the actuator shaft (212, 312).

4. The system (200; 300) according to claim 2 or 3,
**characterized in that**
the first cam (402; 502) and the second cam (404; 504) have the same profile.

5. The system (200; 300) according to any one of the preceding claims,
**characterized in that**
the surgical instrument (202) has a proximal end and a distal end, the distal end of the instrument (202) comprising a first functional distal portion (204) and a second functional distal portion (206) configured to move with respect to one another,
preferably wherein the first and second distal portions (204, 206) of the surgical instrument (202) are connected to one another through a hinge mechanism.

6. The system (200; 300) according to claim 5,
**characterized in that**
the at least one cam (402; 404; 502, 504) comprises:
a first section, **characterized by** a first profile that allows for a large, less precise movement of the first and second distal portions (204, 206) of the surgical instrument (202), and
a second section, **characterized by** a second profile that allows for a precise and strong movement of the first and second distal portions (204, 206) of the surgical instrument (202).

7. The system (200; 300) according to any one of the preceding claims,
**characterized in that**
the at least one movement system (406, 408; 506, 508) at least comprises:
a first roller (406; 506), and
a second roller (408; 508).

8. The system (200; 300) according to claim 7,
**characterized in that**
the first roller (406; 506) is mounted on a first lever (412) and the second roller (408; 508) is mounted on a second lever (414) to control displacement of the first and second distal portions (204, 206) of the surgical instrument (202), respectively.

9. The system (200; 300) according to claim 8,
**characterized in that**
the actuator (310) is configured to actuate movement of the first and second rollers (406, 408; 506; 508) by the same displacement in opposite directions, so that the first and second distal portions (204, 206) of the surgical instrument (202) are correspondingly moved by the same distance in opposite directions, through said first and second levers (412, 414).

10. The system (200; 300) according to any one of claims 7 to 9,
**characterized in that**
the first and second rollers (406, 408; 506; 508) are arranged on opposite sides of the at least one cam (402, 404; 502, 504), preferably in a mirrored position with respect to one another.

11. The system (200) according any one of claims 2 to 10,
**characterized in that**
the cam system (404) further comprises a third cam (416) and a corresponding third roller (418),
wherein the first cam (402), the second cam (404) and the third cam (416) are arranged in different planes,
preferably wherein the third cam (416) has its incline in the opposite direction with respect to the incline of the first and second cams (402, 404).

12. The system (200) according to claim 11,
**characterized in that**
the third roller (418) is arranged in the same plane as the third cam (416).

13. The system (200; 300) according any one of the preceding claims,
**characterized in that**
the system (200; 300) comprises:
a first actuator (310) in combination with first cam system (400; 500), and a second actuator (310) in combination with a second cam system (400; 500).

14. The system (200; 300) according any one of claims 7 to 13,
**characterized in that**
the rotational axis of the at least one cam (402; 404; 502, 504) and the rotational axis (R) of the rollers (406, 408, 418; 506, 508) are parallel each other.

15. The system (200) according any one of the preceding claims,
**characterized in that**
the at least one cam (402, 404) is a radial cam.

16. The system (300) according to any one of claims 1 to 10 and 12 to 14,
**characterized in that**
the at least one cam is a barrel cam.

17. The system (200; 300) according any one of the preceding claims,
**characterized in that**
the at least one actuator (310) is an electric motor.

18. An end-effector system (600) for a surgical, microsurgical or super-microsurgical robot arm, comprising:
the system according to any one of claims 1 to 17;
an and-effector (602), comprising:
an end-effector base,
a first movable finger (604), and
a second movable finger (606),
wherein said first and second movable fingers (604, 606) distally extend from the end-effector base, preferably wherein said first and second movable fingers (604, 606) are connected to one another through a hinge;
a sterile drape (608), configured to cover the end-effector (602), and
a surgical instrument adapter (700), configured to transmit a movement of a tip portion of the first and second movable fingers (604, 606) of the end-effector (602) to the at least one surgical instrument (202) to be operated through the robot arm.

19. A surgical, microsurgical or super-microsurgical robot arm comprising the end-effector system (600) according to claim 18.
